# FASCICULE DE BREVET EUROPEEN

(11) **EP 0 648 201 B1**
(45) Date de publication et mention de la délivrance du brevet: **22.01.1997**
(21) Numéro de dépôt: 93914788.0
(22) Date de dépôt: 30.06.1993
(51) Int. Cl.: C07C 67/31, C07D 307/32, C07C 69/675, C07C 69/732, C07C 69/757, C07B 53/00

(54) **PROCEDE D'HYDROGENATION ENANTIOSELECTIF DU GROUPE CARBONYL, UTILISANT DES COMPLEXES DE RUTHENIUM AVEC BIPHOSPHINE LIGANDS**
VERFAHREN ZUR ENANTIOSELEKTIVEN HYDRIERUNG DER CARBONYLGRUPPE UNTER VERWENDUNG VON RUTHENIUM KOMPLEXEN MIT BIPHOSPHINELIGANDEN
ENANTIOSELECTIVE HYDROGENATION OF THE CARBONYL BOND USING RUTHENIUM COMPLEXES WITH BIPHOSPHINE LIGANDS

(30) Priorité: 02.07.1992 FR 9208160
(43) Date de publication de la demande: 19.04.1995
(73) Titulaire: ELF AQUITAINE, 92400 Courbevoie (FR)
(72) Inventeur: GENET, Jean-Pierre, F-91370 Verrières-Le-Buisson (FR); JUGE, Sylvain, F-91400 Orsay (FR); LAFFITTE, Jean-Alex, F-64000 Pau (FR); PINEL, Catherine, F-78660 Ablis (FR); MALLART, Serge, F-91400 Orsay (FR)
(74) Mandataire: Clisci, Serge
(86) Numéro de dépôt international: FR9300663
(87) Numéro de publication internationale: WO9401390

(56) Documents cités:
- EP-A- 0 158 875
- EP-A- 0 295 109
- EP-A- 0 295 890
- EP-A- 0 339 764
- EP-A- 0 484 271
- WO-A-92/12110
- US-A- 4 343 741

## Description

La présente invention a trait à un nouveau procédé d'hydrogénation catalytique de la double liaison C=O oxo ou cétonique qui est énantiosélectif. Ce nouveau procédé fournit à partir d'une cétone un composé alcool secondaire énantiomériquement pur ou de haute pureté. Il met en oeuvre un composé complexe de ruthénium qui est neutre.

### ART ANTERIEUR

On sait que l'hydrogénation de la fonction carbonyle C=O donne selon les mécanismes 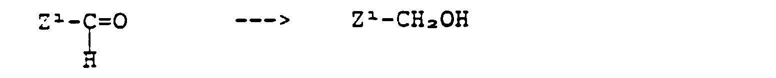 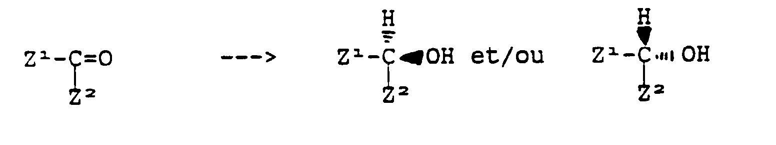 une fonction alcool primaire dépourvue d'atome de carbone asymétrique quand la fonction carbonyle considérée est constituée d'un groupe formyle, d'une part, et une fonction alcool secondaire comportant un atome de carbone asymétrique quand la fonction carbonyle considérée est constituée d'un groupe oxo (i.e. cétonique), d'autre part. D'une manière générale, l'hydrogénation d'une double liaison C=O cétonique donne un composé à fonction alcool secondaire qui est constitué d'un mélange des énantiomères, ce mélange est ensuite traité selon une méthode connue en soi, en général longue et fastidieuse, pour isoler l'énantiomère jugé intéressant, notamment par chromatographie chirale (en particulier par HPLC) ou cristallisation fractionnée.

Pour pallier aux inconvénients de ce type d'isolation, on sait que l'on a déjà proposé par le passé d'utiliser, en tant que catalyseurs d'hydrogénation énantiosélectifs de la fonction carbonyle C=O cétonique, des composés complexes de ruthénium comprenant notamment des groupements acétate ou halogénure. Voir à cet effet les publications de POWEL et al., J. Chem. Soc., (1968), pages 159-161, R. NOYORI et al., J. Am. Chem. Soc., (1987), pages 5856-5858, M. KITAMURA et al., J. Am. Chem. Soc., (1988), 110, pages 629-631, et T. IKARIYA et al., J. Chem. Soc., Chem. Commun., (1985), page 922.

Dans le domaine de l'hydrogénation de composés organiques éthylèniquement insaturés présentant au moins une double liaison C=C aliphatique, on sait que l'on a déjà utilisé des catalyseurs à base de ruthénium appartenant à l'ensemble des composés complexes dits "ioniques", d'une part, et dits "neutres", d'autre part.

On connaît notamment de la demande de brevet français No 90 165 414 déposée le 28 décembre 1992 (publication sous le numéro FR-A-2 671 079 le 3 juillet 1992) et de la demande internationale correspondante PCT/FR 91/01077 déposée le 27 décembre 1991, pour l'hydrogénation catalytique de composés éthylèniquement insaturés, des catalyseurs qui sont des composés complexes de ruthénium "neutres" et dans lesquels (i) deux groupes phospbine tertiaire sont liés au même atome de Ru, les atomes de P desdits groupes phosphine tertiaire étant reliés entre eux par une chaine comportant au moins deux atomes caténaires, et (il) deux restes allyle ou métallyle sont liés au même atome de Ru.

Les complexes de Ru selon ladite demande française et ladite demande internationale, qui sont des composés diallyliques de (diphosphino)ruthénium, peuvent être représentés par la formule I suivante 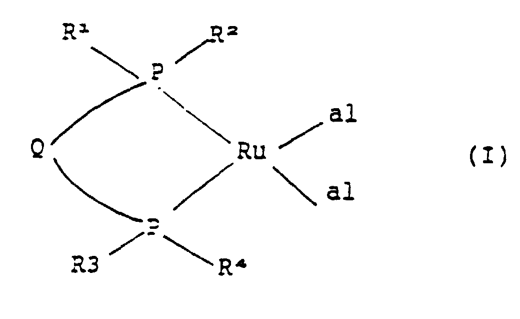 dans laquelle
- al: représente un groupe allylique,
- Q: représente un pont hydrocarboné comportant au moins deux atomes de carbone caténaires et susceptible de contenir un à quatre hétéroatomes caténaires choisis parmi O, S, N et Si,
- R¹ à R⁴: qui peuvent être identiques ou différents, représentent chacun un groupe alkyle en C₁-C₁₈, cycloalkyle en C₅-C₇ ou aryle en C₆-C₁₂.

Les groupes allyliques ai précités comprennent ici tous les groupes allyle. Les plus simples et les plus économiques étant plus précisément le groupe allyle proprement dit de formule CH₂CH=CH₂ et surtout le groupe méthallyle de formule CH₂C(CH₃)=CH₂ qui est désigné ci-après en abrégé "Met".

### BUTS DE L'INVENTION

Un des buts de l'invention est de disposer d'un nouveau procédé d'hydrogénation catalytique de la double liaison C=O cétonique sans affecter de façon substantielle le groupement carbonyle C=O inclus dans tout groupe acide carboxylique -C(=O)-OH, carboxylate -C(=O)-O- ou carboxamide -C(=O)-NH- éventuellement présent dans le composé cétone que l'on veut réduire en composé alcool.

Un autre but de l'invention est de disposer d'un nouveau procédé d'hydrogénation catalytique de la double liaison C=O cétonique permettant d'obtenir des composés du type alcool secondaire optiquement actifs avec des rendements supérieurs à 85 %, de préférence supérieurs à 90 % et mieux de l'ordre de 99-100 % par rapport aux composés cétoniques de départ, d'une part, et des e.e d'au moins 35 %, d'autre part.

Selon l'invention, on se propose également de préparer des composés du type alcool secondaire optiquement actifs en vue de les utiliser comme intermédiaires de synthèse dans la préparation de produits énantiomériquement purs qui sont utiles dans d'autres domaines, notamment en thérapeutique.

### OBJET DE L'INVENTION

Les buts précités et d'autres avantages sont obtenus au moyen de la présente invention. Plus précisément, l'invention dans son aspect le plus large vise une nouvelle utilisation des complexes de Ru précités de formule I pour l'hydrogénation catalytique de la double liaison C=O cétonique, alors que ces dits complexes n'avaient été initialement préconisés que pour l'hydrogénation catalytique de la double liaison C=C de composés éthylèniquement insaturés selon la demande internationale PCT/FR 91/01077 sus-visée (cette dernière étant incorporée ici à titre de référence).

Selon l'invention, l'on préconise donc un nouveau procédé d'hydrogénation catalytique énantiosélectif de la double liaison C=O cétonique en fonction alcool secondaire, ledit procédé, qui met en oeuvre l'utilisation d'un complexe neutre de ruthénium en tant que catalyseur et d'un composé à fonctionnalité C=O en tant que substrat à hydrogéner, étant caractérisé en ce qu'il comprend l'hydrogénation de la fonctionnalité carbonyle cétonique du substrat dans un solvant approprié au moyen de H₂ (de préférence, sous une pression de 10⁵ Pa à 1,5 x 10⁷ Pa, à une température de -20 à 150°C , en présence de 0,01 à 10 % molaires, par rapport audit substrat), d'un composé diallylique de (diphosphino)ruthénium répondant à la formule 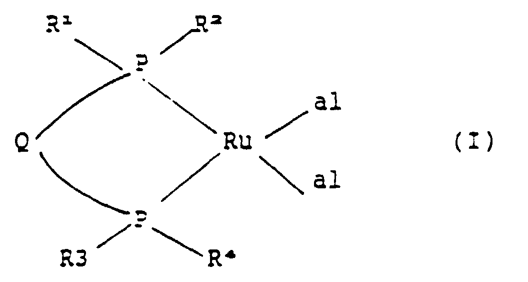 dans laquelle
- al: représente un groupe allylique choisi de préférence parmi CH₂CH=CH₂ et CH₂C(CH₃)=CH₂,
- Q: représente un pont hydrocarboné comportant au moins deux atomes de carbone caténaires et susceptible de contenir un à quatre hétéroatomes caténaires choisis parmi O, S, N et Si,
- R¹ à R⁴: qui peuvent être identiques ou différents, représentent chacun un groupe hydrocarboné, éventuellement substitué, choisi parmi les groupes alkyle en C₁-C₁₈, cycloalkyle en C₅-C₇ et aryle en C₆-C₁₂.

Ce procédé est particulièrement intéressant dans le cas de l'hydrogénation de substrats tels que (i) les dicétones et (ii) les composés comprenant au moins une onction cétone et au moins une autre fonction, notamment les composés alpha- et béta-cétoesters.

Selon l'invention, l'on préconise également l'utilisation de ce procédé pour l'obtention de composés à fonction alcool secondaire particuliers, optiquement actifs et utiles comme intermédiaires dans la synthèse stéréospécifique d'autres produits notamment intéressants en thérapeutique tant humaine que vétérinaire.

### ABREVIATIONS

Par commodité, les abréviations suivantes ont été utilisées dans la présente description.
- al =: allyle ou méthallyle
- BDPP =: 2,4-bis(diphénylphosphino)pentane 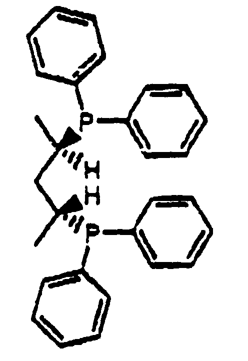
- BINAP =: 2,2'-bis(diphénylphosphino)-1,1'-binaphtyle 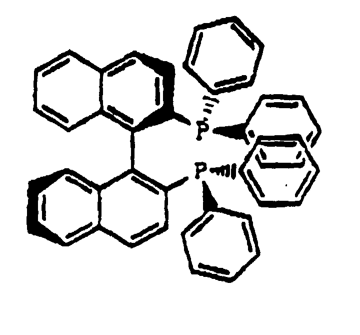
- Boc =: t-butyloxycarbonyle
- bNp =: bétanaphthyle (i.e. 2-naphtyle)
- BNPE =: bis(naphthyl phényl phosphino)éthane 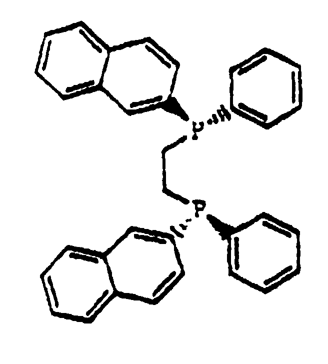
- BNPPMDS =: bis(2-naphthyl phényl phosphino-méthano) diphénylsilane 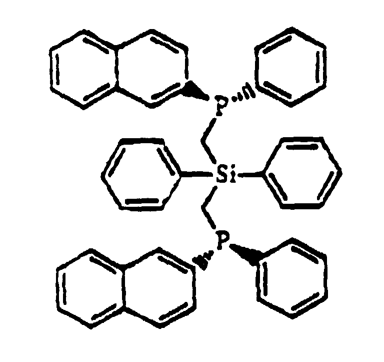
- BPPM =: N-Boc-4-diphenylphosphino-2-(diméthylphosphinométhyl)-pyrrolidine 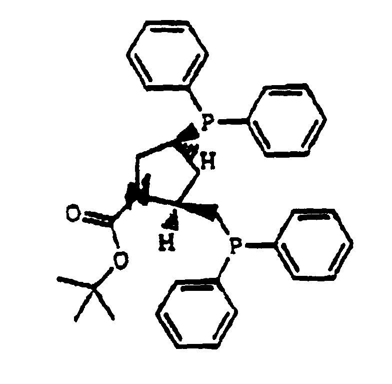
- Bu =: n-butyle
- Bz =: benzyle
- CHIRAPHOS =: 2,3-bis(diphénylphosphino)butane 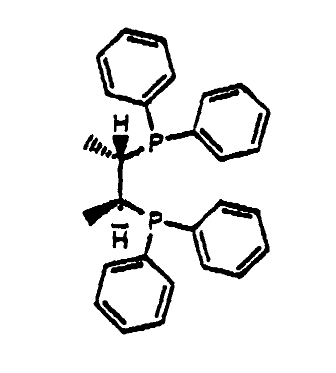
- DIOP =: 0,0-isopropylidène-2,3-dihydroxy-1,4-bis (diphénylphosphino)butane 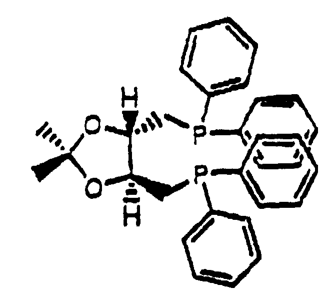
- DIPAMP =: 1,2-bis(phényl o-anisyl phosphino)éthane 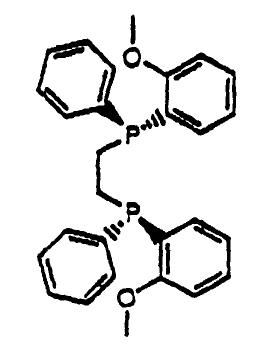 qui est également dénommé (R,R)-1,2-bis(phényl ortho-anisylphosphino)éthane
- Et =: éthyle
- HPLC =: chromatographie liquide de haute performance
- i-Pr =: isopropyle
- Me =: méthyle
- MeO =: méthoxy
- Met =: méthallyle [i.e. CH₂C(CH₃)=CH₂]
- Ph =: phényle
- Pr =: n-propyle
- RT =: température ambiante (15-20°C)
- s-Bu =: sec.-butyle
- t-Bu =: tert.-butyle
- THF =: tétrahydrofuranne

### DESCRIPTION DETAILLEE DE L'INVENTION

Comme indiqué ci-dessus, les groupes R¹, R², R³ et R⁴, identiques ou différents, représentent chacun un groupe hydrocarboné, éventuellement substitué, choisi parmi les groupes alkyle en C₁-C₁₈, cycloalkyle en C₅-C₇ et aryle en C₆-C₁₂. De façon avantageuse, les groupes R¹, R², R³ et R⁴, identiques ou différents, représenteront chacun un groupe alkyle en C₁-C₈ (notamment méthyle, éthyle, isopropyle, propyle, s-butyle, i-butyle, t-butyle 2,2-diméthylpropyle ou 1,1, 3,3-tétraméthylbutyle), un groupe cycloalkyle en C₅-C₇ (notamment cyclopentyle ou mieux cyclohexyle) ou un groupe aryle en C₆-C₁₂ (notamment phényle, tolyle, xylyle, halogénophényle, p-méthoxyphényle, p-éthoxyphényle, p-(t-butyloxy) phényle ou naphtyle).

De préférence, R¹, R², R³ et R⁴, identiques ou différents, représenteront chacun un groupe cyclohexyle, phényle, phényle substitué en position para par un groupe alkyle en C₁-C₄, phényle substitué en position para par un groupe alkoxy en C₁-C₄, phényle substitué en position para par un groupe F, Cl ou Br, ou 2-naphtyle.

La chaîne hydrocarbonée du pont Q est soit une chaîne hydrocarbonée insaturée, soit une chaîne hydrocarbonée saturée. En particulier, ledit pont Q pourra avoir l'une des structures suivantes

(a) -(CH₂)ₘ-,

et

(b) -(CH₂)ₘ-A-(CH₂)ₚ-

dans lesquelles
n, m et p, identiques ou différents, représentent chacun un nombre entier de 1 à 6,
A représente O, S, PR, SiR₂ ou NR, où R est alkyle en C₁-C₄, cycloalkyle en C₅₋ C₆, aryle en C₆-C₁₀, benzyle ou phénéthyle.

Quand le pont Q renfermera de 2 à 4 atomes caténaires, on pourra avoir notamment l'une des structures suivantes : 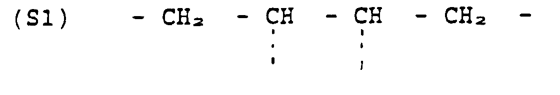 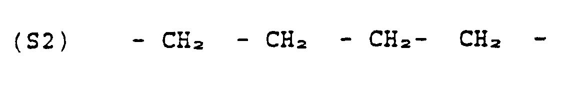 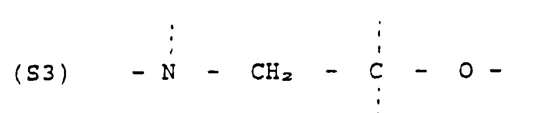 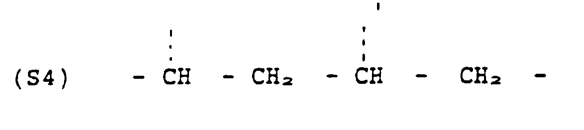 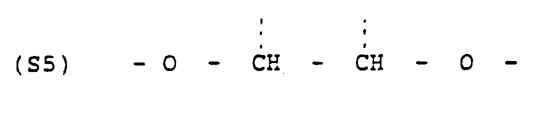 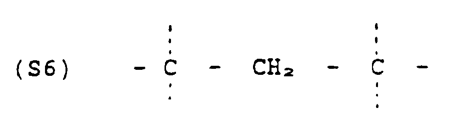 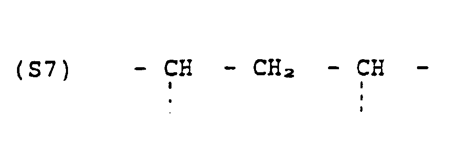 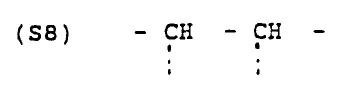 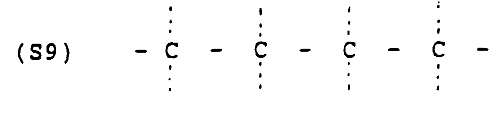 ou

(S10) -CH(CH₃)-CH(CH₃)-

dans lesquels les traits en pointillé désignent chacun un substituant différent de H, voire encore une double liaison C=C sur deux atomes de carbone vicinaux éventuellement inclus dans un cycle ; ainsi la structure (S9) ci-desssus du pont Q pourra être :

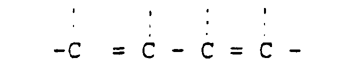

Eu égard à ces définitions, ledit pont Q pourra être notamment choisi parmi l'ensemble constitué par

(a1) -CH₂-,

(a2) -CH₂CH₂-,

(b1) -CH₂-O-CH₂-,

(b2) -CH₂-S-CH₂-,

(b3) -CH₂-P(Ph)-CH₂-,

(b4) -CH₂-Si(Me)₂-CH₂-,

(b5) -CH₂-Si(Ph)₂-CH₂-,

(b6) -CH₂-Si(Bz)₂-CH₂-,

(b7) -CH₂-Si(Et)₂-CH₂-

ou

(b8) -CH₂CH₂-P(Ph)-CH₂CH₂.

De façon avantageuse, le catalyseur selon l'invention aura pour formule

L*Ru(Z)₂ (II)

où
Z est Met, et
L* est un ligand chiral du type diphosphino où les deux atomes de phosphore sont reliés entre eux par un pont hydrocarboné comportant au moins deux atomes de carbone caténaires et susceptible de contenir 1 à 4 hétéroatomes caténaires choisis parmi O, S, N et Si.

Dans le cas de la formule II, le ligand chiral L* représente le système divalent R¹R²P-Q-PR³R⁴ (de la formule I) dans lequel R¹ à R⁴ sont avantageusement cnacun un groupe aryle (de préférence Ph ou bNp) éventuellement substitué ou un groupe cyclohexyle, et Q est un pont hydrocarboné dont la chaîne caténaire peut être partiellement ou totalement incluse dans un cycle.

Parmi les ligands chiraux L* qui conviennent selon l'invention, on peut notamment citer les ligands BDPP, BINAP, BNPE, BNPPMDS, BPPM, CHIRAPHOS, DIOP et DIPAMP visés ci-dessus dans le chapitre "ABREVIATIONS", ainsi que les ligands analogues notamment ceux déjà décrits dans la littérature, en particulier les ligands BPPM, CyDIOP, CyPRONOP, Cy-cy CAPP, CyPOP AE, DEGUPHOS, DPCB, NORPHOS, PNNP, PROPHOS et SKEWPHOS qui sont dans leur majorité illustrés dans la demande internationale PCT/FR 91/01077 précitée.

Les complexes "neutres" de ruthénium de formule II que l'on préfère selon l'invention pour l'hydrogénation catalytique éniantosélective des composés à double liaison C=O cétonique, sont ceux dans lesquels le ligand chiral L* est DIOP ou BINAP.

Les composés chiraux de formules I et II, qui sont des complexes diallyliques de (diphosphino)ruthénium, peuvent être préparés selon une méthode connue en soi.

La méthode que l'on préconise pour leur préparation est celle décrite dans la demande internationale PCT/FR 91/01077 précitée.

Le procédé d'hydrogénation de l'invention comprend le traitement d'un substrat, ici un composé à fonctionnalité carbonyle cétonique, avec H₂, dans un solvant inerte approprié, en présence d'un composé diallylique de (diphosphino)ruthénium de formule I ou mieux de formule II en tant que catalyseur d'hydrogénation catalytique, dans les conditions préférentielles suivantes :
- température comprise entre -20 et +150°C,
- pression comprise entre 10⁵ et 1,5x10⁷ Pa, et
- rapport molaire catalyseur/substrat compris entre 0,01/100 et 10/100.

On préfère plus particulièrement que la température de la réaction d'hydrogénation soit supérieure ou égale à 15°C. En pratique, l'hydrogénation catalytique selon l'invention sera réalisée à une température de 15 à 100°C sous une pression de H₂ de l'ordre de 5x10⁵ à 10⁷ Pa.

De façon encore plus avantageuse, le procédé d'hydrogénation catalytique de la double liaison C=O cétonique de l'invention sera réalisé dans les conditions suivantes :
- température comprise entre 25-50°C,
- pression H₂ comprise entre 5x10⁶ et 10⁷ Pa, et
- rapport molaire catalyseur/substrat compris 0,5/100 et 4/100.

La durée d'hydrogénation n'est pas critique. Elle sera en général notamment supérieure ou égale à 1h. En fonction du substrat et du catalyseur, elle pourra par exemple être notamment comprise entre 10 et 70h.

Parmi les solvants appropriés qui conviennent, on peut notamment citer les alcools tels que MeOH, EtOH et PrOH anhydres, les hydrocarbures aromatiques tels que les benzène, toluène et xylène, les hydrocarbures aliphatiques tels que les pentane, hexane, heptane et similaires, les hydrocarbures halogénés tels que les CH₂Cl₂, ClCH₂-CH₂Cl, et Cl₂CH-CHCl₂, les éthers, notamment les éthers cycliques tels que le THF, et leurs mélanges. Les solvants préférés selon l'invention sont le toluène et CH₂Cl₂.

Lors de la mise en oeuvre de la réaction d'hydrogénation selon le procédé de l'invention, l'on recommande d'utiliser le substrat à une concentration, dans le solvant, de 0,2 à 1,5 mole/litre, et de préférence de 0,4 à 0,8 mole/litre.

Par ailleurs, l'on peut ajouter dans le milieu réactionnel une faible quantité d'amine tertiaire, notamment Et₃N pour améliorer le rendement dans des cas très particuliers, en particulier quand le solvant du milieu réactionnel est MeOH. Cependant un tel ajout n'est pas essentiel, il peut même constituer une gêne dans l'obtention du (R)-2-chloromandélate de méthyle, à partir du (2-chlorophényl)-oxoacétate de méthyle, ainsi que cela a été illustré ci-après. Néanmoins, dans les cas où cet ajout est effectué, l'on introduira ladite amine tertiaire dans le milieu réactionnel selon une quantité correspondante à (i) un rapport pondéral amine tertiaire/solvant de l'ordre de 1/100, ou (ii) un rapport molaire amine tertiaire/substrat analogue ou identique au rapport molaire catalyseur/substrat.

### MEILLEUR MODE

Le meilleur mode pour mettre en oeuvre le procédé de l'invention consiste, en particulier pour l'obtention du (R)-2-chloromandélate de méthyle à partir du (2-chlorophényl)oxoacétate de méthyle, à soumettre à une hydrogénation le substrat cétonique devant être réduit, cette hydrogénation étant effectuée dans un solvant choisi parmi les CH₂Cl₂ et toluène, en présence d'un catalyseur de formule II où L* est DIOP ou BINAP, dans les conditions suivantes :
- température comprise entre 25-50°C,
- pression H₂ comprise entre 5x10⁶ et 10⁷ Pa,
- rapport molaire catalyseur/substrat de l'ordre de 1/100, et
- concentration du substrat comprise entre 0,4 et 0,8 mole/litre.

D'autres avantages et caractéristiques de l'invention seront mieux compris à la lecture qui va suivre d'exemples d'hydrogénation et d'essais comparatifs. Bien entendu, l'ensemble de ces éléments n'est nullement limitatif mais est donné à titre d'illustration. En particulier, l'homme du métier par des essais-simples peut déterminer, sans sortir du cadre de la présente invention, (i) le catalyseur chiral de formule I ou mieux de formule II (notamment par le choix du ligand L*), (ii) le solvant et (iii) les conditions opératoires, qui conviennent le plus favorablement à l'hydrogénation d'un substrat cétonique donné.

### MODE OPERATOIRE

### Réduction de la double liaison C=O cétonique au moyen d'un complexe de ruthénium

Dans un tube réactionnel contenant 6x10⁻⁶ mole d'un catalyseur de formule I selon l'invention, on ajoute dans 2 ml de solvant, une quantité de 5,9x10⁻⁴ mole de substrat alpha- cétoester à l'aide d'un dispositif "PIPETMAN GIBSON". Le tube est ensuite placé sous argon dans un réacteur puis on purge trois fois avec H₂ ledit réacteur. Le milieu réactionnel est maintenu sous vive agitation magnétique (pendant au moins 1 h, en particulier pendant 10 à 64 h selon les exemples donnés ci-après), à une température et une pression déterminées. A la fin de la réaction, le solvant est évaporé sous vide (trompe à eau). Un liquide foncé visqueux est obtenu. C'est ce mode opératoire A qui a été utilisé dans les exemples qui suivent, avec le cas échéant des modifications en ce qui concerne la nature du substrat et/ou les quantités de catalyseur et de substrat.

### EXEMPLE 1

### Obtention de (R)-pantolactone

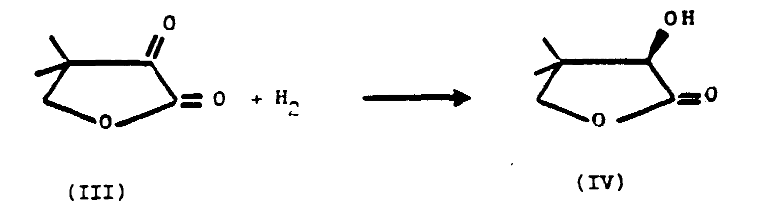

Le composé (R)-pantolactone de formule IV, qui répond à la nomenclature systématique de L-dihydro-3-hydroxy-4H-diméthyl-2(3H)-furanone ou L-3-hydroxy-4,4-diméthyl-tétrahydrofuran-2-one, est un énantiomère utile dans la synthèse de l'énantiomère acide L-(+)-pantothénique, ce dernier étant le seul isomère de l'acide pantothénique à avoir une activité vitaminique.

On procède à l'hydrogénation catalytique du composé de formule III (substrat carbonyle), qui a pour nomenclature systématique 4,4-diméthyl-tétrahydrofuran-2,3-dione, en présence de DIPAMP Ru (Met)₂ et de Et₃N, dans les conditions suivantes :
- température : 50°C,
- pression H₂ : 1,013x10⁷Pa (100 atm),
- rapport molaire : DIPAMP Ru (Met)₂/substrat : 1/100,
- durée : 64 h,
- solvant : toluène,
- concentration du substrat : 0,5 mole/l,
- rapport molaire : Et₃N/substrat : 1/100.

On obtient l'énantiomère de formule IV avec un rendement de 100 % (calculé à partir du substrat de départ) et un excès énantiomérique (e.e) de 14 % . **EXEMPLES 2-4 ET EXEMPLE COMPARATIF CP 1**

Obtention de (R)-4-chloro-3-hydroxybutanoate d'éthyle précurseur de la carnitine (vitamine BT, agent pharmacologique important, responsable du métabolisme humain et du transport des acides gras à travers les parois mitochondriales).

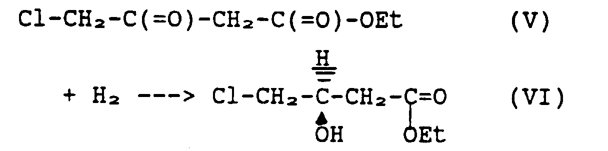

L'hydrogénation catalytique du substrat V pour obtenir l'énantiomère VI a été réalisée dans les conditions suivantes :
- catalyseur : BINAP Ru (Met)₂, BINAP Ru (Br)₂ ou CHIRAPHOS Ru (Met)₂,
- température : 80 ou 90°C,
- pression H₂ : 10⁷ Pa,
- rapport molaire catalyseur/substrat : 0,1/100 ou 1/100,
- durée : 10 h,
- solvant : MeOH,
- concentration du substrat : 0,5 mole/ml.

Les conditions opératoires, qui ont été suivies, sont consignées dans le tableau I ci-après, avec les rendements et e.e qui ont été mesurés.

Ces résultats mettent en évidence que le catalyseur BINAP Ru (Met)₂ donne des rendements et e.e supérieurs à ceux de BINAP Ru (Br)₂, et que le catalyseur CHIRAPHOS Ru (Met)₂ est ici peu énantiosélectif. **EXEMPLES 5-6 ET EXEMPLES COMPARATIFS CP 2-CP 3**

### Obtention du (R)-2-chloromandélate de méthyle

On a effectué la réaction suivante : 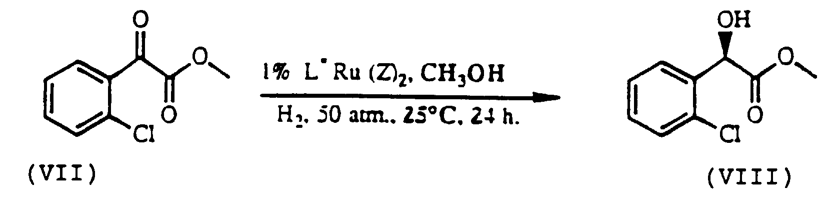 pour l'obtention du (R)-2-chloromandélate de méthyle à partir du (2-chlorophényl)oxoacétate de méthyle de formule VII dans les conditions suivantes :
- catalyseur : voir tableau II,
- température : 25°C,
- pression H₂ : 5,065x10⁶ Pa (50 atm),
- rapport molaire catalyseur/substrat : 1/100,
- durée : 24 h,
- solvant : MeOH,
- concentration du substrat : 0,5 mole/ml.

Les rendements et e.e, qui ont été obtenus, sont consignés dans le tableau II ci-après.

**EXEMPLES 7-8 ET EXEMPLE COMPARATIF CP 4**

Obtention du (R)-2-chloromandélate de méthyle

On a obtenu l'énantiomère de formule VIII en procédant comme indiqué dans les exemples 5-6 ci-dessus en remplaçant le méthanol par le toluène en tant que solvant.

Les résultats obtenus figurent dans le tableau III ci-après.

### EXEMPLES 9-10

### Obtention du (R)-2-chloromandélate de méthyle

On a obtenu l'énantiomère de formule VIII en procédant comme indiqué dans les exemples 5-6 ci-dessus en remplaçant le méthanol par un autre solvant (soit MeOH + 1 % Et₃N, soit THF).

Les résultats obtenus figurent dans le tableau IV ci-après.

### EXEMPLES 11-13 ET EXEMPLE COMPARATIF CP 5

### Obtention du (R)-2-chloromandélate de méthyle

On a obtenu l'énantiomère de formule VIII en procédant comme indiqué dans les exemples 5-6 ci-dessus en remplaçant le méthanol par le dichlorométhane en tant que solvant.

Les résultats obtenus figurent dans le tableau V ci-après.

### EXEMPLES 14-16 ET EXEMPLES COMPARATIFS CP 6-CP 7

### Obtention du (R)-2-chloromandélate de méthyle

On procède comme indiqué dans les exemples 11-13 ci-dessus, avec la différence que la réaction est réalisée à 50°C au lieu de 25°C.

Les résultats obtenus figurent dans le tableau VI ci-après.

### EXEMPLES 17-21

### Obtention du (R)-2-chloromandélate de méthyle

On procède comme indiqué dans les exemples 14-16 ci-dessus en faisant varier la nature du solvant et/ou le rapport molaire catalyseur/substrat.

Les résultats qui ont été obtenus sont consignés dans le tableau VII ci-après.

L'ensemble des résultats figurant dans les tableaux II-VII montre que les meilleurs catalyseurs pour l'obtention du (R)-2-chloromandélate de méthyle à partir du (2-chlorophényl)oxoacétate de méthyle ont été les catalyseurs chiraux BINAP Ru (Met)₂ et DIOP Ru (Met)₂.

Ces catalyseurs ont été avantageusement utilisés dans un rapport molaire catalyseur/substrat de 0,5/100 à 4/100. Les meilleurs solvants ont été les CH₂Cl₂ et toluène, sans ajout de Et₃N.

### EXEMPLE 22

### Application à la préparation de dérivés du tétralol, fort utiles dans l'industrie pharmaceutique.

On hydrogène une tétralone suivant la réaction : 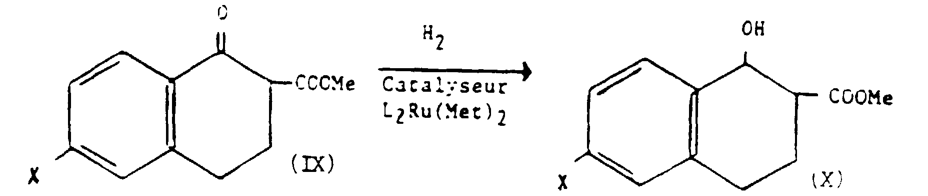 X pouvant être H, MeO, EtO, etc.

On a opéré dans CH₂Cl₂ à 80°C sous 10⁷ Pa pendant 120 heures. Trois préparations, effectuées chacune avec 2 % de l'un des catalyseurs suivants, ont conduit à des rendements quantitatifs :
(a) catalyseur [DIOP]Ru(Met)₂ - rendement 100 %
(b) catalyseur [BINAP]Ru(Met)₂ - rendement 100 %
(c) catalyseur [CHIRAPHOS]Ru(Met)₂ - rendement 100 %
X étant ici MeO dans chacune des trois préparations.

### EXEMPLE 23

Préparation d'un autre composé intermédiaire, d'intérêt pharmaceutique, d' α-chloro-hydroxyacétate d'éthyle à partir de la cétone correspondante, suivant la réaction :

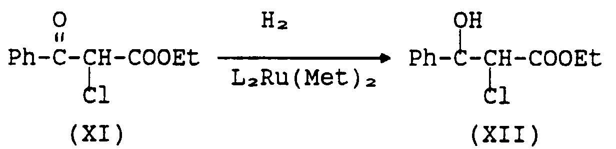

Le composé de formule XII est obtenu selon cette réaction à partir d'un α-chloro-acétoacétate d'éthyle de formule XI par hydrogénation énantiosélective en présence de 2 % de catalyseur, ici le [BINAP]Ru(Met)₂, dans CH₂Cl₂ à 50°C pendant 60 heures sous 7 x 10⁶ Pa ; le rendement est de 100 % et l'excès énantiomérique ee de 70 % pour la forme anti, et de 43 % pour la forme syn (rapport syn/anti : 56/44).

Le procédé de l'exemple 23 est applicable à la préparation de composés α-chloro-hydroxyacétate d'alkyle (où le groupe alkyle, notamment en C₁-C₆ est différent de Et).

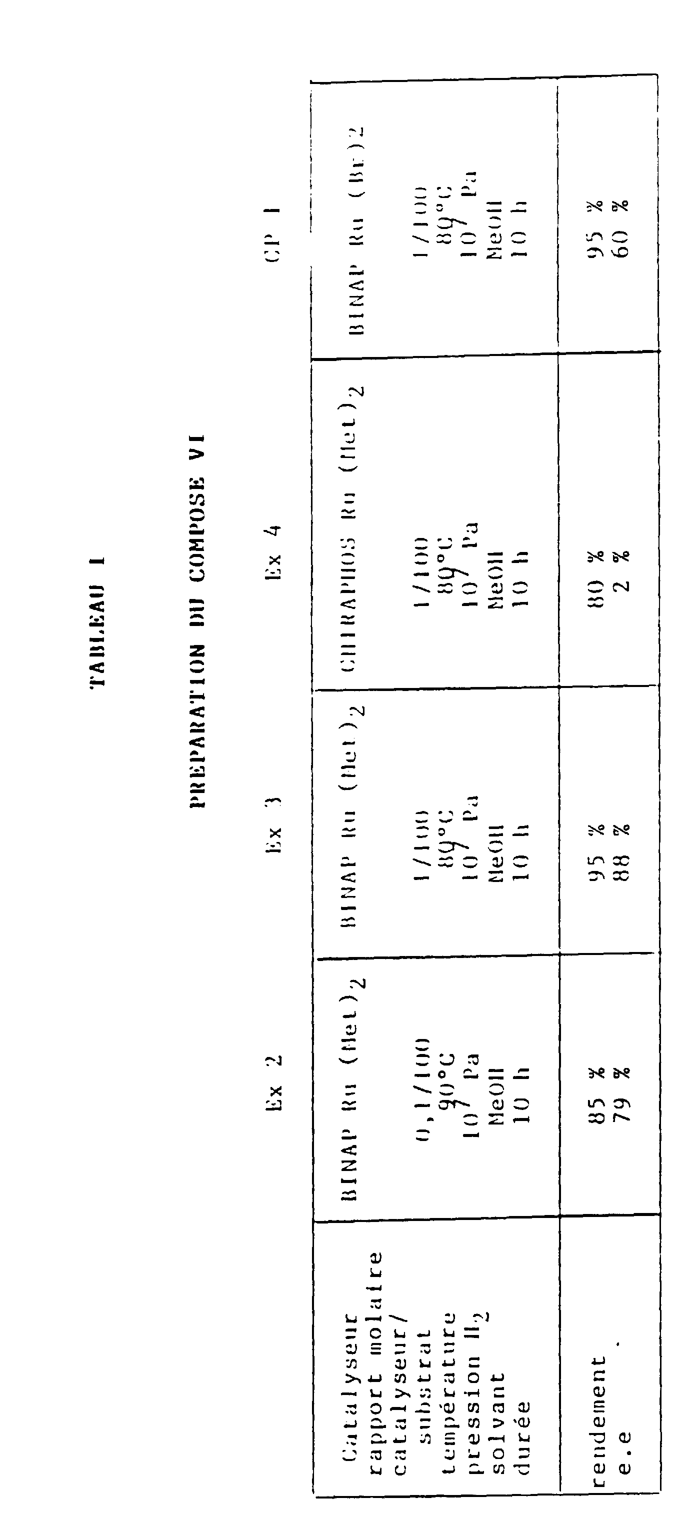

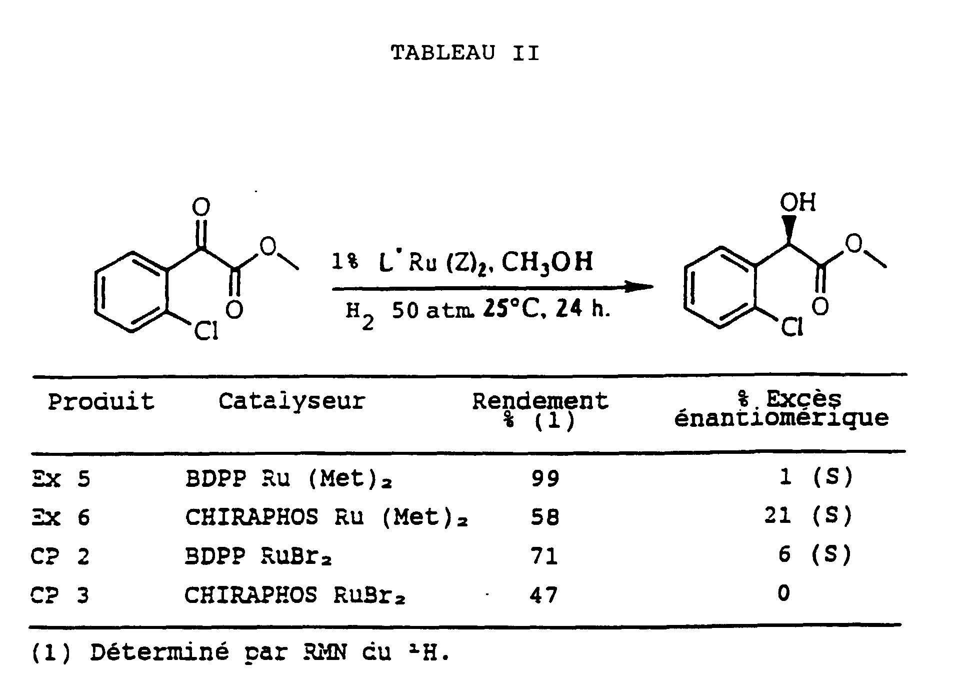

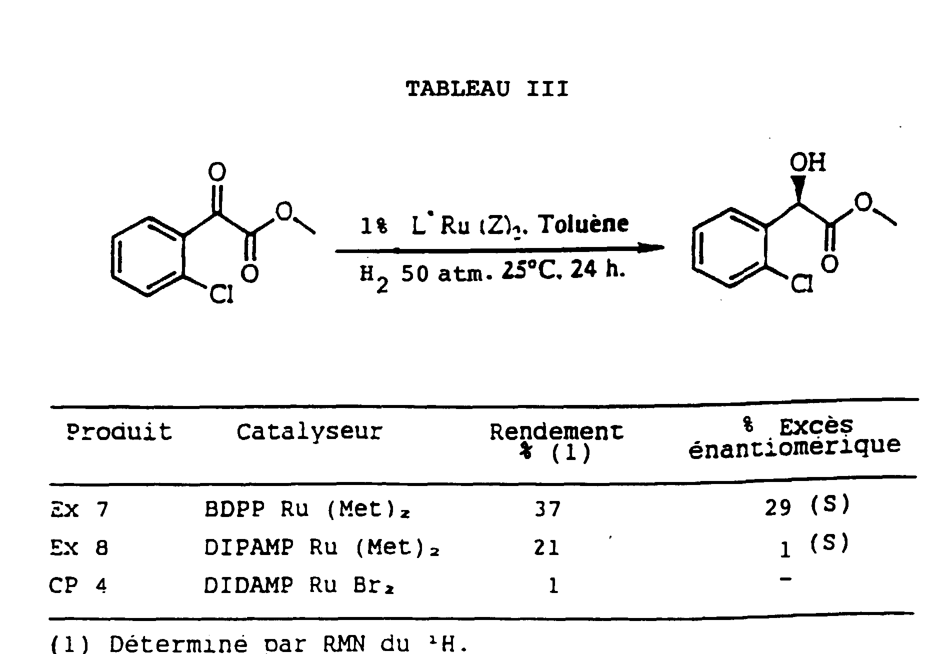

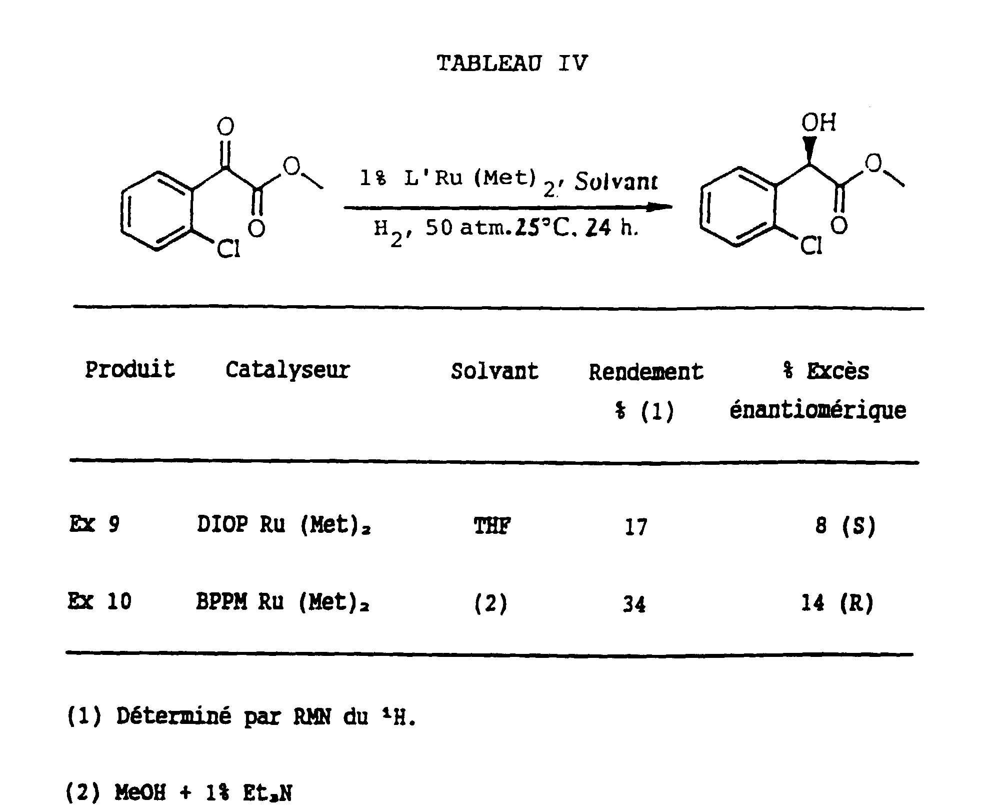

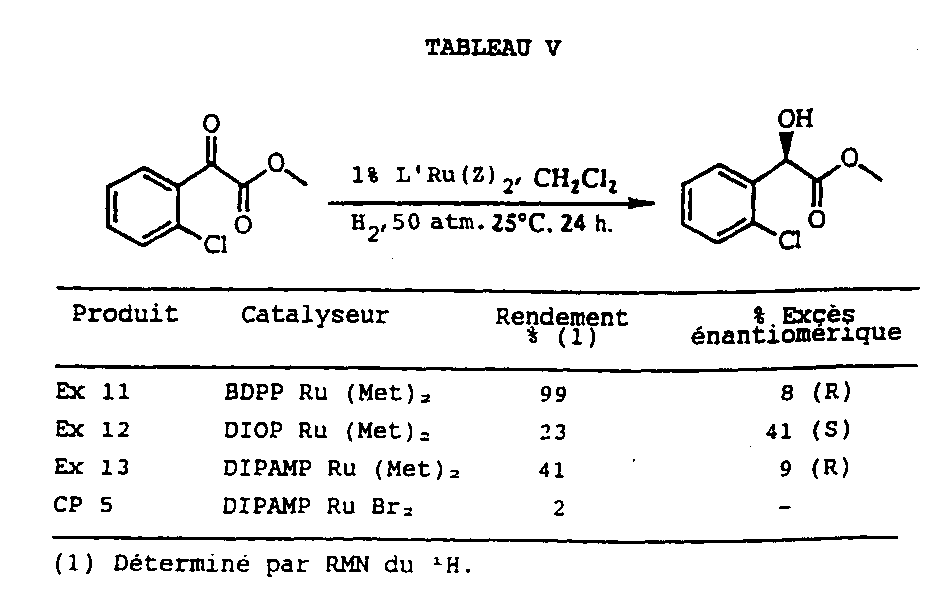

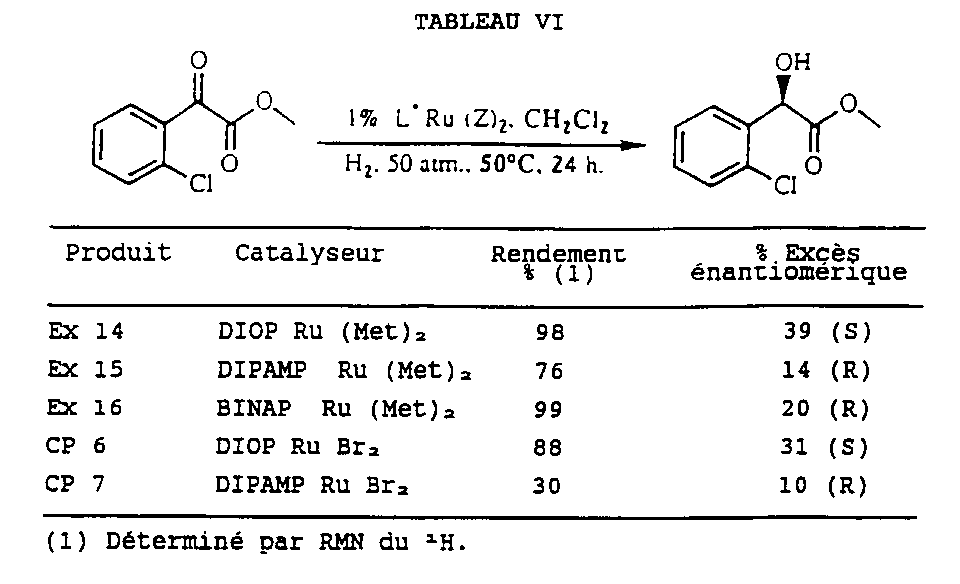

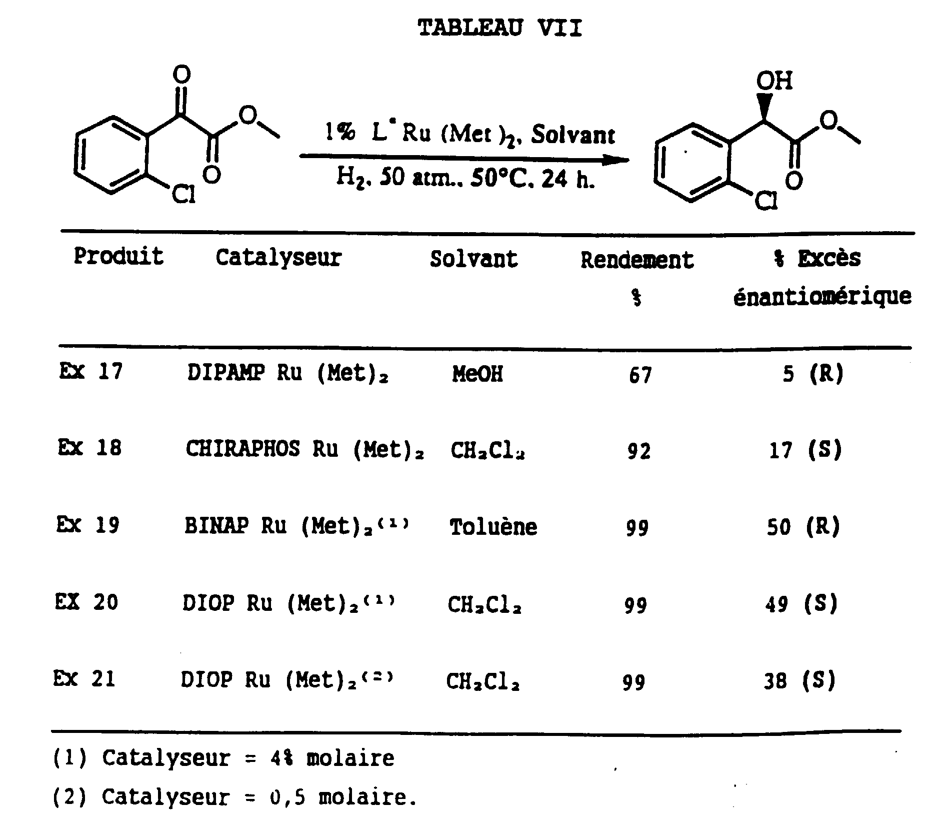

## Revendications

1. Procédé d'hydrogénation catalytique énantiosélectif de la double liaison C=O cétonique en fonction alcool secondaire, ledit procédé, qui met en oeuvre l'utilisation d'un complexe neutre de ruthénium en tant que catalyseur et à un composé à fonctionnalité C=O en tant que substrat à hydrogéner, étant caractérisé en ce qu'il comprend l'hydrogénation de la fonctionnalité carbonyle cétonique du substrat au moyen de H₂, en présence d'un composé diallylique de (diphosphino)ruthénium répondant à la formule 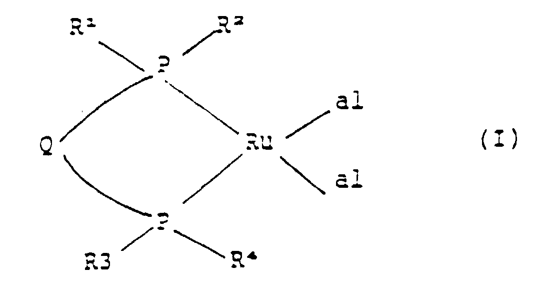 dans laquelle
al représente un groupe allylique choisi de préférence parmi CH₂CH=CH₂ et CH₂C(CH₃)=CH₂,
Q représente un pont hydrocarboné comportant au moins deux atomes de carbone caténaires et susceptible de contenir un à quatre hétéroatomes caténaires choisis parmi O, S, N et Si,
R¹ à R⁴ qui peuvent être identiques ou différents, représentent chacun un groupe hydrocarboné, éventuellement substitué, choisi parmi les groupes alkyle en C₁-C₁₈, cycloalkyle en C₅-C₇ et aryle en C₆-C₁₂.

2. Procédé suivant la revendication 1, dans lequel le groupe R¹R²P-Q-PR³R⁴ est choisi parmi l'ensemble constitué par les ligands chiraux BDPP, BINAP, BNPE, BNPPMDS, BPPM, CHIRAPHOS, DIOP, DIPAMP BPPM, CyDIOP, CyPRONOP, Cy-cy CAPP, CyPOP AE, DEGUPHOS, DPCB, NORPHOS, PNNP, PROPHOS et SKEWPHOS.

3. Procédé suivant la revendication 1, dans lequel on utilise un solvant choisi parmi l'ensemble constitué par les MeOH, EtoH, PrOH, benzène, toluène, xylène, pentane, hexane, heptane, THF, CH₂Cl₂, ClCH₂-CH₂Cl, Cl₂CH-CHCl₂ et leurs mélanges.

4. Procédé suivant la revendication 1, dans lequel le catalyseur répond la formule
L*Ru(Z)₂ (II)
où
Z est méthallyle, et
L* est un ligand chiral du type diphosphino où les deux atomes de phosphore sont reliés entre eux par un pont hydrocarboné comportant au moins deux atomes de carbone caténaires et susceptible de contenir à à hétéroatomes caténaires choisis parmi O, S, N et Si.

5. Procédé suivant la revendication 4, dans lequel L* est un ligand chiral BDPP, BINAP, BNPE, BNPPMDS, BPPM, CHIRAPHOS, DIOP ou DIPAMP.

6. Procédé suivant la revendication 1 ou 4, dans lequel l'hydrogénation est réalisée dans les conditions suivantes :
- température comprise entre -20 et +150°C,
- pression H₂ comprise entre 10⁵ et 1,5x10⁷ Pa, et
- rapport molaire catalyseur/substrat compris entre 0,01/100 et 10/100.

7. Procédé suivant la revendication 1 ou 4, dans lequel l'hydrogénation est réalisée dans les conditions suivantes :
- température comprise entre 15-100°C,
- pression H₂ comprise entre 5x10⁵ et 10⁷ Pa,
- rapport molaire catalyseur/substrat compris entre 0,5/100 et 4/100, et
- concentration du substrat comprise entre 0,4 et 0,8 mole/litre.

8. Procédé suivant la revendication 4, dans lequel l'hydrogénation est effectuée dans un solvant choisi parmi les CH₂Cl₂ et toluène, en présence d'un catalyseur de formule II où L* est DIOP ou BINAP, dans les conditions suivantes :
- température comprise entre 25-50°C,
- pression H₂ comprise entre 5x10⁶ et 10⁷ Pa,
- rapport molaire catalyseur/substrat de l'ordre de 1/100, et
- concentration du substrat comprise entre 0,4 et 0,8 mole/litre.

9. Procédé suivant l'une quelconque des revendications 1 à 8, caractérisé en ce que substrat à double liaison C=0 cétonique est choisi parmi l'ensemble des composés alpha- et béta-cétoesters.

10. Utilisation du procédé suivant l'une quelconque des revendications 1 à 8, pour l'obtention d'un isomère énantiomériquement pur choisi parmi l'ensemble constitué par les
(R)-pantolactone,
(R)-4-chloro-3-hydroxybutanoate d'éthyle, et
(R)-2-chloromandélate de méthyle.

11. Procédé suivant une des revendications 1 à 9, appliqué à la préparation d'un dérivé de tétralol.

12. Procédé suivant une des revendications 1 à 9, appliqué à la préparation d'un α-chloro-hydroxyacétate d'alkyle.

## Patentansprüche

1. Verfahren zur katalytischen enantioselektiven Hydrierung der ketonischen C=O-Doppelbindung zu einer sekundären Alkoholfunktion, wobei das Verfahren, das einen neutralen Rutheniumkomplex als Katalysator und eine Verbindung mit C=O-Funktionalität als Hydrierungssubstrat verwendet, dadurch gekennzeichnet ist, daß es die Hydrierung der ketonischen Carbonylfunktion des Substrates mittels H₂ in Gegenwart einer (Diphosphino)-rutheniumdiallyl-Verbindung umfaßt, die der Formel 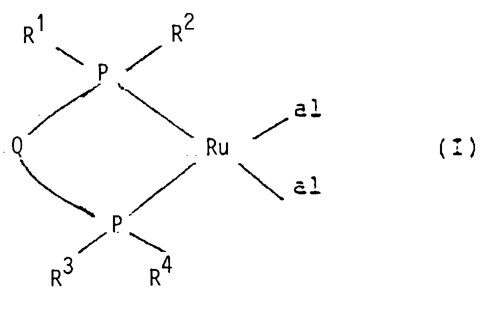 entspricht, in der
al für eine Allylgruppe steht, vorzugsweise ausgewählt aus CH₂CH=CH₂ und CH₂C(CH₃)=CH₂,
Q eine Kohlenwasserstoffbrücke darstellt, die aus wenigstens 2 Kettenkohlenstoffen besteht und 1 bis 4 Kettenheteroatome aufweisen kann, ausgewählt aus O, S, N und Si,
R¹ bis R⁴, die gleich oder verschieden sein können, jeweils eine Kohlenwasserstoffgruppe darstellen, die gegebenenfalls substituiert ist, ausgewählt aus den Gruppen C₁-C₁₈-Alkyl, C₅-C₇-Cycloalkyl und C₆-C₁₂-Aryl.

2. Verfahren nach Anspruch 1, bei dem die Gruppe R¹R²P-Q-PR³R⁴ ausgewählt wird aus der Gesamtheit, bestehend aus den chiralen Liganden BDPP, BINAP, BNPE, BNPPMDS, BPPM, CHIRAPHOS, DIOP, DIPAMP BPPM, CyDIOP, CyPRONOP, Cy-Cy CAPP, CyPOP AE, DEGUPHOS, DPCB, NORPHOS, PNNP, PROPHOS und SKEWPHOS.

3. Verfahren nach Anspruch 1, bei dem ein Lösungsmittel eingesetzt wird, ausgewählt aus der Gesamtheit, bestehend aus MeOH, EtOH, PrOH, Benzol, Toluol, Xylol, Pentan, Hexan, Heptan, THF, CH₂Cl₂, ClCH₂-CH₂Cl, Cl₂CH-CHCl₂ und ihren Gemischen.

4. Verfahren nach Anspruch 1, bei dem der Katalysator der Formel
L*Ru(Z)₂ (II)
entspricht, worin
Z für Methallyl steht, und
L* ein chiraler Ligand vom Typ Diphosphino ist, worin die beiden Phosphoratome miteinander über eine Kohlenwasserstoffbrücke verbunden sind, die aus wenigstens 2 Kettenkohlenstoffatomen besteht und 1 bis 4 Kettenheteroatome enthalten kann, ausgewählt aus O, S, N und Si.

5. Verfahren nach Anspruch 4, bei dem L* ein chiraler BDPP-, BINAP-, BNPE-, BNPPMDS-, BPPN-, CHIRAPHOS-, DIOP- oder DIPAMP-Ligand ist.

6. Verfahren nach Anspruch 1 oder 4, bei dem die Hydrierung unter den folgenden Bedingungen durchgeführt wird:
- Temperatur zwischen -20 und +150 °C,
- H₂-Druck zwischen 10⁵ und 1,5 x 10⁷ Pa und
- Molverhältnis Katalysator/Substrat zwischen 0,01/100 und 10/100.

7. verfahren nach Anspruch 1 oder 4, bei dem die Hydrierung unter den folgenden Bedingungen durchgeführt wird:
- Temperatur zwischen 15 bis 100 °C,
- H₂-Druck zwischen 5 x 10⁵ und 10⁷ Pa,
- Molverhältnis Katalysator/Substrat zwischen 0,5/100 und 4/100 und
- Substratkonzentration zwischen 0,4 und 0,8 mol/Liter.

8. Verfahren nach Anspruch 4, bei dem die Hydrierung in einem Lösungsmittel, ausgewählt aus CH₂Cl₂ und Toluol, in Gegenwart eines Katalysators der Formel II, worin L* für DIOP oder BINAP steht, unter den folgenden Bedingungen durchgeführt wird:
- Temperatur zwischen 25 bis 50 °C,
- H₂-Druck zwischen 5 x 10⁶ und 10⁷ Pa,
- Molverhältnis Katalysator/Substrat in der Größenordnung von 1/100, und
- Substratkonzentration zwischen 0,4 und 0,8 mol/Liter.

9. Verfahren nach einem der Ansprüche 1 bis 8, dadurch gekennzeichnet, daß das Substrat mit der ketonischen C=O-Doppelbindung aus der Gesamtheit der α- und β-Ketoesterverbindungen ausgewählt wird.

10. Anwendung des Verfahrens nach einem der Ansprüche 1 bis 8, um ein Isomer enantiomer rein zu erhalten, ausgewählt aus der Gesamtheit, bestehend aus
(R)-Pantolacton,
(R)-4-Chlor-3-hydroxyethylbutanoat und
(R)-2-Chlormethylmandelat.

11. Verfahren nach einem der Ansprüche 1 bis 9, das zur Herstellung eines Tetralol-Derivates angewendet wird.

12. Verfahren nach einem der Ansprüche 1 bis 9, das zur Herstellung eines α-Chlorhydroxyalkylacetates angewendet wird.

## Claims

1. A process for enantioselective catalytic hydrogenation of the ketone C=O double bond to a secondary alcohol function, said process using a neutral ruthenium complex as the catalyst and a compound containing a C=O group as the substrate to be hydrogenated, characterised in that it comprises hydrogenation of the carbonyl ketone function of the substrate using H₂, in the presence of a diallyl (diphosphino) ruthenium compound with formula 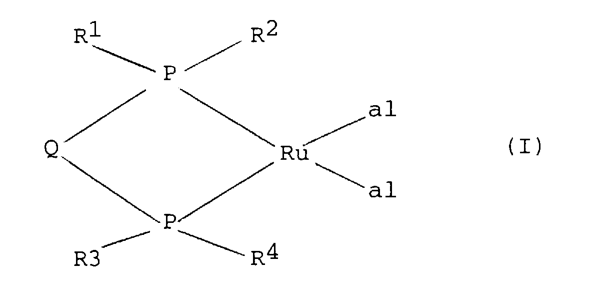 where
al represents an allyl group, preferably CH₂CH=CH₂ or CH₂C(CH₃)=CH_{2,}
Q represents a hydrocarbon bridge containing at least two catenary carbon atoms and which can contain one to four catenary heteroatoms selected from O, S, N and Si,
R¹ to R⁴ may be identical or different and each represents a hydrocarbon group which may be substituted, selected from C₁-C₁₈ alkyl, C₅-C₇ cycloalkyl and C₆-C₁₂ aryl groups.

2. A process according to claim 1 wherein the R¹R²P-Q-PR³R⁴ group is selected from the group constituted by chiral ligands BDPP, BINAP, BNPE, BNPPMDS, BPPM, CHIRAPHOS, DIOP, DIPAMP, BPPM, CyDIOP, CyPRONOP, Cy-cy CAPP, CyPOP AE, DEGUPHOS, DPCB, NORPHOS, PNNP, PROPHOS and SKEWPHOS.

3. A process according to claim 1 wherein a solvent is used which is selected from the group constituted by MeOH, EtOH, PrOH, benzene, toluene, xylene, pentane, hexane, heptane, THF, CH₂Cl₂, ClCH₂-CH₂Cl, Cl₂CH-CHCl₂ and mixtures thereof.

4. A process according to claim 1 wherein the catalyst has formula
L*Ru(Z)₂ (II)
where
Z is methallyl, and
L* is a chiral diphosphino type ligand where the two phosphorus atoms are bonded to each other by a hydrocarbon bridge containing at least two catenary carbon atoms which can contain one to four catenary heteroatoms selected from O, S, N and Si.

5. A process according to claim 4 wherein L* is a BDPP, BINAP, BNPE, BNPPMDS, BPPM, CHIRAPHOS, DIOP or DIPAMP chiral ligand.

6. A process according to claim 1 or claim 4 wherein hydrogenation is carried out under the following conditions:
- a temperature of between -20°C and +150°C,
- a pressure of H₂ between 10⁵ Pa and 1.5 x 10⁷ Pa, and
- a catalyst/substrate molar ratio of between 0.01/100 and 10/100.

7. A process according to claim 1 or claim 4 wherein hydrogenation is carried out under the following conditions:
- a temperature of 15°C to 100°C,
- a pressure of H₂ between 5 x 10⁵ Pa and 10⁷ Pa,
- a catalyst/substrate molar ratio of between 0.5/100 and 4/100, and
- a substrate concentration of between 0.4 mole/litre and 0.8 mole/litre.

8. A process according to claim 4 wherein hydrogenation is carried out in a solvent selected from CH₂Cl₂ and toluene in the presence of a catalyst with formula II where L* is DIOP or BINAP under the following conditions:
- a temperature of 25°C to 50°C,
- a pressure of H₂ between 5 x 10⁶ Pa and 10⁷ Pa,
- a catalyst/substrate molar ratio in the order of 1/100, and
- a substrate concentration of between 0.4 mole/litre and 0.8 mole/litre.

9. A process according to any one of claims 1 to 8 characterised in that the substrate containing the ketone C=O double bond is selected from alpha- and beta-ketoesters.

10. Use of a process according to any one of claims 1 to 8 to obtain an enantiomerically pure isomer selected from the group constituted by
(R)-pantolactones,
ethyl (R)-4-chloro-3-hydroxybutanoates, and
methyl (R)-2-chloromandelates.

11. A process according to any one of claims 1 to 9 applied to the preparation of a tetralol derivative.

12. A process according to any one of claims 1 to 9 applied to the preparation of an alkyl α-chlorohydroxyacetate.
